# EUROPÄISCHE PATENTSCHRIFT

(11) **EP 0 305 322 B1**
(45) Veröffentlichungstag und Bekanntmachung des Hinweises auf die Patenterteilung: **16.03.1994**
(21) Anmeldenummer: 88730193.5
(22) Anmeldetag: 27.08.1988
(51) Int. Cl.: C07D 471/04, A61K 31/435

(54) **Isoxazol-Beta-carbolin-Derivate**
Isoxazole-beta-carboline derivatives
Dérivés d'isoxazole-bêta-carboline

(30) Priorität: 28.08.1987 DK 4498/87; 09.09.1987 DE 3730667
(43) Veröffentlichungstag der Anmeldung: 01.03.1989
(73) Patentinhaber: SCHERING AKTIENGESELLSCHAFT, 13342 Berlin (DE)
(72) Erfinder: Huth, Andreas, Dr., D-1000 Berlin 38 (DE); Rahtz, Dieter, Dr., D-1000 Berlin 38 (DE); Rohde, Ralph, Dr., D-1000 Berlin 45 (DE); Schmiechen, Ralph, Dr., D-1000 Berlin 42 (DE); Seidelmann, Dieter, Dr., D-1000 Berlin 41 (DE); Schneider, Herbert Hans, Dr., D-1000 Berlin 15 (DE); Stephens, David Norman, Dr., D-1000 Berlin 31 (DE); Hansen, John Bondo, Dr., DK-2800 Lyngby (DK); Engelstoft, Mogens, DK-2500 Vaerlose (DK); Olsen, Preben, Kopenhagen NV (DK)

(56) Entgegenhaltungen:
- EP-A- 0 054 507
- EP-A- 0 161 574

## Beschreibung

Die Erfindung betrifft neue β-Carbolin-Derivate, die in 3-Stellung mit Isoxazol-oder Isoxazolin-Derivaten substituiert sind, das Verfahren zu ihrer Herstellung und ihre Verwendung als Arzneimittel.

In der EP-A 54 507 werden β-Carboline beschrieben, die in 3-Stellung mit dem Isoxazolon-Rest substituiert sind. Diese Verbindungen zeigen geringe Affinität zu den Benzodiazepin-Rezeptoren.

Es wurde nun gefunden, daß die erfindungsgemäßen in 3-Stellung mit Isoxazol- oder Isoxazolin-resten substituierten β-Carboline überraschenderweise eine hohe spezifische Affinität zu Benzodiazepin-Rezeptoren zeigen, indem sie radioaktiv markiertes Flunitrazepam von den Benzodiazepin-Rezeptoren verdrängen.

Die erfindungsgemäßen Verbindungen haben die allgemeine Formel I 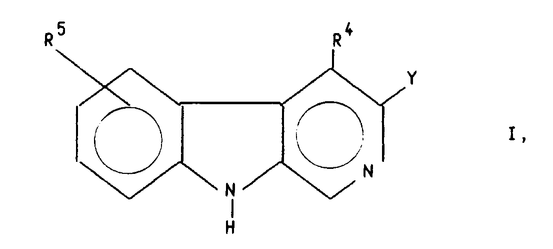
worin
Y den Rest 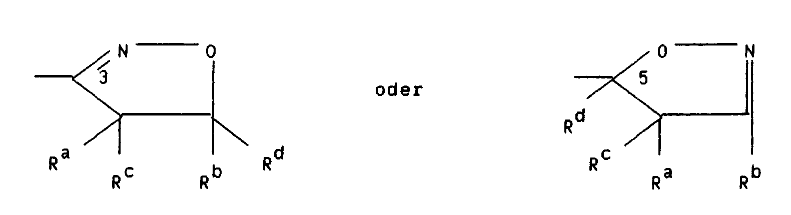
darstellt und
R^{a} und R^{b} gleich oder verschieden sind und jeweils Wasserstoff, C₁₋₆-Alkoxy, C₃₋₇-Cycloalkyl, Phenyl, gegebenenfalls mit Hydroxy, C₁₋₆-Alkoxy, Phenyl oder Halogen substituiertes C₁₋₆-Alkyl oder C₁₋₆-Alkoxycarbonyl darstellen und R^{c} und R^{d} gleich oder verschieden sind und jeweils Wasserstoff oder C₁₋₆-Alkyl oder gemeinsam eine Bindung bedeuten und
R⁴ Wasserstoff, C₁₋₆-Alkyl oder C₁₋₆-Alkoxy-C₁₋₆-Alkyl ist und
R⁵ Wasserstoff, Halogen, OR⁶, NR⁷R⁸ oder CH R⁹R¹⁰ ist
mit R⁶ in der Bedeutung von C₁₋₆-Alkyl, C₃₋₇ Cycloalkyl, eines gegebenenfalls mit Halogen, C₁₋₄-Alkoxy, C₁₋₄-Alkyl oder Amino ein- oder zweifach substituierten C₇₋₁₀ -Aralkylreste, eines gegebenenfalls mit Halogen, NO₂, Cyano, C₁₋₄-Alkyl oder C₁₋₄-Alkoxy ein- bis zweifach substituiertes C₆₋₁₀-Arylrestes, eines gegebenenfalls mit Halogen, NO₂, Cyano, C₁₋₄-Alkyl oder C₁₋₄-Alkoxy ein- bis dreifach substituierten Sechsring-Heteroaromaten mit ein- bis zwei Stickstoffatomen oder eines gegebenenfalls mit Halogen, NO₂, Cyano, C₁₋₄-Alkyl oder C₁₋₄-Alkoxy ein- bis dreifach substituierten Fünfringheteroaromaten mit ein- bis zwei Sauerstoff-, Schwefel- und/oder Stickstoffatomen, R⁷ und R⁸ sind gleich oder verschieden und stellen Wasserstoff, C₁₋₆-Alkyl, C₃₋₆-Alkenyl oder gemeinsam mit dem Stickstoffatom einen gegebenenfalls mit ein bis zwei C₁₋₄-Alkylgruppen substituierten Pyrrolidin-, Piperidin-, Morpholin-, Piperazin- oder Thiomorpholin-Ringdar, R⁹ in der Bedeutung von Wasserstoff oder C₁₋₆-Alkyl, R¹⁰ in der Bedeutung von Wasserstoff, C₁₋₆-Alkyl, OR¹¹ oder NR¹²R¹³ mit R¹¹ in der Bedeutung von C₁₋₆ Alkyl, R¹² und R¹³ sind gleich oder verschieden und stellen Wasserstoff, C₁₋₆-Alkyl oder gemeinsam mit dem Stickstoffatom einen gegebenenfalls mit ein bis zwei C₁₋₄-Alkylgruppen substituierten Pyrrolidin-, Piperidin-, Morpholin-, Piperazin- oder Thiomorpholin-Ringdar und der Substituent R⁵ ein- oder zweifach steht, wobei, falls R⁴ und R⁵ Wasserstoff bedeuten, Y nicht 3-Methyl-isoxazol-5-yl sein kann.

Der Substituent R⁵ kann in 5-, 6-, 7- und/oder 8-Stellung ein- oder zweifach stehen, wobei die Substitution in 5- oder 6-Stellung bevorzugt ist.

Unter C₁₋₆-Alkyl ist jeweils eine gerade oder verzweigte Alkylgruppe mit 1 bis 6 Kohlenstoffen zu verstehen, beispielsweise seien Methyl, Ethyl, Propyl, Isopropyl, Butyl, sek. Butyl, tert. Butyl, Isobutyl, Pentyl und Hexyl genannt, wobei C₁₋₄-Alkyle als bevorzugt anzusehen sind.

Bedeutet R⁴ eine Alkoxyalkylgruppe, so ist C₁₋₄-Alkoxy-C₁₋₂-Alkyl als bevorzugt zu betrachten.

Als Cycloalkylrest R^{a}, R^{b} und R⁶ seien beispielsweise Cyclopropyl, Cyclobutyl, Cyclopentyl, Cyclohexyl, Cycloheptyl genannt.

Als Substituenten der Alkylreste R^{a} und R^{b} kommen Hydroxy, C₁₋₆-Alkoxy, vorzugsweise C₁₋₄-Alkoxy, Phenyl oder Halogen in Betracht, insbesondere seien für substituierte Alkylreste R^{a} und R^{b} CH₂OH, CH₂-0-C₁₋₄-Alkyl und Benzyl genannt.

Unter Halogen ist immer Fluor, Chlor, Brom oder Iod zu verstehen, wobei Chlor und Brom bevorzugt sind.

Der Aralkylrest R⁶ kann 7-10 Kohlenstoffatome haben und im Alkylrest gerade oder verzweigt sein. Bevorzugt sind Ar-C₁₋₂-Alkyle, die im Arylrest gegebenenfalls ein- oder zweifach substituiert sein können.

Geeignete Substituenten in Aralkylrest sind beispielsweise Halogen, C₁₋₄-Alkoxy, C₁₋₄-Alkyl oder Amino.

Bevorzugt sind Ar-C₁₋₂-Alkyle, die im Arylrest durch 1 bis 2 Halogene substituiert sein können wie beispielsweise Benzyl, Phenethyl, α-Methylbenzyl, 4-Chlorphenethyl, 3-Brombenzyl u.a.

Der Arylrest R⁶ kann 6-10 Kohlenstoffatome haben und gegebenenfalls ein- bis zweifach substituiert sein beispielsweise mit Halogen, Nitro, Cyano, C₁₋₄-Alkyl oder C₁₋₄-Alkoxy.

Als bevorzugt seien Phenyl, 2-Chlorphenyl, 4-Chlorphenyl, 2,4-Dichlorphenyl, 2-Nitrophenyl und 2-Cyanophenyl genannt.

Bedeutet R⁶ einen heteroaromatischen Rest, so kann dieser 5- oder 6-gliedrig und gegebenenfalls ein- bis dreifach substituiert sein.

Geeignete Substituenten des Hetarylrestes sind die für den Arylrest R⁶ genannten Substituenten.

Der Heteroaromat kann ein bis zwei Heteroatome wie Schwefel, Stickstoff und/oder Sauerstoff enthalten.

Bevorzugt sind Sechsring-Heteroaromaten mit ein bis zwei Stickstoffatomen und Fünfringheteroaromaten mit ein bis zwei Sauerstoff-, Schwefel-und/oder Stickstoffatomen, die durch Halogen substituiert sein können, wie beispielsweise Pyridin, Pyrimidin, Pyrazin, Pyridazin, Furan, Thiophen, Pyrrol, Imidazol, Thiazol. Insbesondere seien als bevorzugte Reste Pyridin, Pyrimidin, Pyridazin, Pyrazin, und 5-Brom-pyridin genannt.

Bilden R⁷, R⁸ und R¹², R¹³ gemeinsam mit dem Stickstoffatom einen gegebenenfalls ein weiteres Heteroatom enthaltenden gesättigten heterocyclischen Fünf- oder Sechsring, so stellt dieser beispielsweise Pyrrolidin, Piperidin, Morpholin, Piperazin oder Thiomorpholin dar und kann gegebenenfalls mit ein bis zwei C₁₋₄-Alkylgruppen substituiert sein wie beispielsweise 2,6-Dimethylmorpholin oder N-Methylpiperazin.

Als Alkenylreste R⁷ und R⁸ seien beispielsweise Allyl, Butenyl genannt.

Die neuen Verbindungen der allgemeinen Formel I sind pharmakologisch wirksame Substanzen, die sich unter anderem durch eine Wirkung auf das Zentralnervensystem auszeichnen und insbesondere als Psychopharmaka für die Humanmedizin geeignet sind. Da die erfindungsgemäßen Verbindungen nicht nur eine hohe spezifische Affinität zu den Benzodiazepin-Rezeptoren sondern auch eine sehr niedrige Toxizität besitzen, weisen sie einen besonders günstigen therapeutischen Index auf. Gleichzeitig ist die metabolische Stabilität gegenüber den bekannten β-Carbolinen deutlich verbessert. Auf Grund der überraschend guten Wirksamkeit im PTZ-Krampftest eignen sich die erfindungsgemäßen Verbindungen insbesondere zur Behandlung von Epilepsie und Angst.

Die erfindungsgemäßen Verbindungen können zur Formulierung von pharmazeutischen Präparationen, zum Beispiel für die orale und parenterale Anwendung nach an sich bekannten Methoden der Galenik verwendet werden.

Als Hilfsstoffe zur Formulierung von pharmazeutischenm Präparationen sind solche physiologisch verträglichen organischen und anorganischen Trägersubstanzen für die enterale und parenterale Anwendung geeignet, die gegenüber den erfindungsgemäßen Verbindungen inert sind.

Als Trägersubstanzen seien beispielsweise genannt: Wasser, Salzlösungen, Alkohole, Polyäthylenglykole, polyhydroxyethoxyliertes Rizinusöl, Gelatine, Lactose, Amylose, Magnesiumstearat, Talkum, Kieselsäure, Fettsäuremono- und diglyceride, Pentaerythritolfettsäureester, Hydroxymethylcellulose und Polyvinylpyrrolidon.

Die pharmazeutischen Präparationen können sterilisiert und/oder mit Hilfsstoffen, wie Schmiermitteln, Konservierungsstoffen, Stabilisatoren, Netzmitteln, Emulgatoren, Puffermitteln und Farbstoffen, versetzt werden.

Für die parenterale Anwendung sind insbesondere Injektionslösungen oder Suspensionen, insbesondere wässrige Lösungen der aktiven Verbindungen in polyhydroxyethoxyliertem Rizinusöl, geeignet.

Als Trägersysteme können auch grenzflächenaktive Hilfsstoffe wie Salze der Gallensäuren oder tierische oder pflanzliche Phospholipide, aber auch Mischungen davon sowie Liposome oder deren Bestandteile verwendet werden.

Für die orale Anwendung sind insbesondere Tabletten, Dragees oder Kapseln mit Talkum und/oder Kohlenwasserstoffträger oder -binder, wie zum Beispiel Lactose, Mais- oder Kartoffelstärke, geeignet. Die Anwendung kann auch in flüssiger Form erfolgen, wie zum Beispiel als Saft, dem gegebenenfalls ein Süßstoff beigefügt wird.

Die erfindungsgemäßen Verbindungen werden in einer Dosiseinheit von 0,05 bis 100 mg aktiver Substanz in einem physiologisch verträglichen Träger eingebracht.

Die erfindinngsgemäßen Verbindungen werden in einer Dosis von 0,1 bis 300 mg/Tag, vorzugsweise 0,1 bis 30 mg/Tag, besonders bevorzugt 1-20 mg/Tag angewendet.

Die Herstellung der erfindungsgemäßen Verbindungen erfolgt nach an sich bekannten Methoden.

Beispielsweise erfolgt die Herstellung der Verbindungen der allgemeinen Formel I, indem man
a) Nitriloxide der allgemeinen Formel II 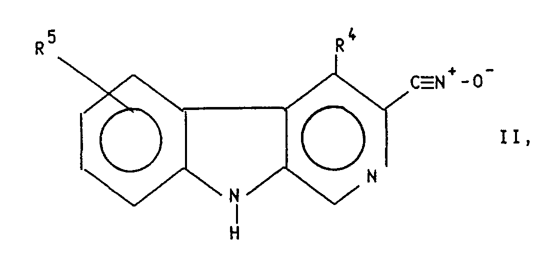 worin
   R⁴ und R⁵ die oben genannte Bedeutung haben und der Stickstoff in 9-Stellung geschützt sein kann, mit einer Verbindung der allgemeinen Formel III 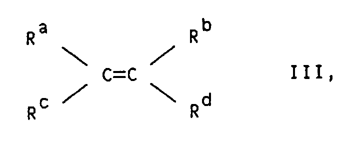 worin R^{a}, R^{b}, R^{c} und R^{d} die obige Bedeutung haben, zu Verbindungen der allgemeinen Formel I mit Y in der Bedeutung 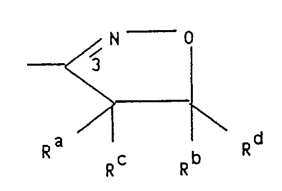 mit R^{a}, R^{b}, R^{c}, und R^{d} in der obigen Bedeutung bei Temperaturen von 0° - 40° C cyclisiert, oder
b) Nitriloxide der allgemeinen Formel IV

   R^{b} - C≡N⁺ - O⁻ IV,

   worin R^{b} die obige Bedeutung hat, mit Verbindungen der allgemeinen Formel V 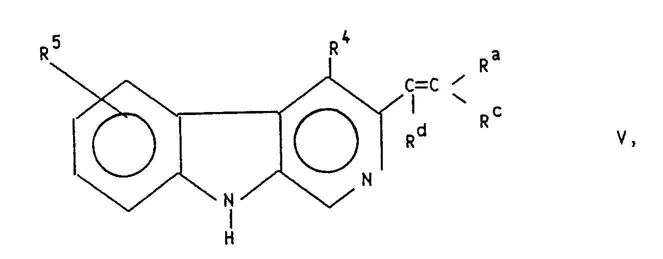 worin
   R^{a}, R^{c}, R^{d}, R⁴ und R⁵ die obige Bedeutung haben, zu Verbindungen der allgemeinen Formel I mit Y in der Bedeutung 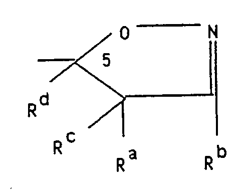 bei Temperaturen von 0° - 40° C cyclisiert, oder
c) Verbindungen der allgemeinen Formel VI 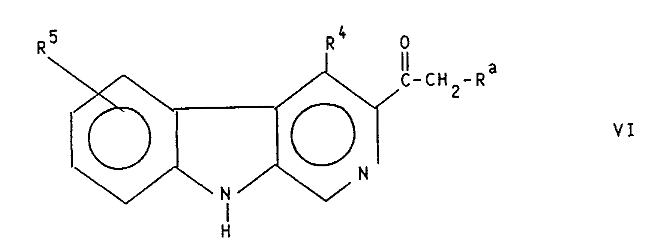 worin
   R^{a}, R⁴ und R⁵ die obige Bedeutung haben, zum Enaminon umsetzt und mit Hydroxylamin-O-sulfonsäure bei Raumtemperatur bis zu 100° C cyclisiert zu Verbindungen der allgemeinen Formel I mit Y in der Bedeutung 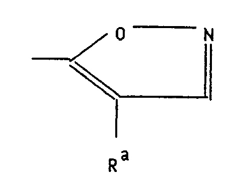 worin R^{a} die obige Bedeutung hat.

Die Cycloaddition der Verbindungen der allgemeinen Formel II und IV erfolgt bei Temperaturen von 0-40°C in einem aprotischen Lösungsmittel und ist im allgemeinen nach 4-20 Stunden beendet. Als aprotische Lösungsmittel sind aliphatische und cyclische Ether wie Diethylether, Tetrahydrofuran, Dioxan, halogenierte Kohlenwasserstoffe wie Dichlorethan, Methylenchlorid, Chloroform, Kohlenwasserstoffe wie Hexan, Pentan und Dimethylformamid, Dimethylsulfoxid, u.a. geeignet.

Sind die Ausgangsverbindungen gasförmig wie beispielsweise Acetylen, so ist es vorteilhaft, die entsprechenden flüssigen Verbindungen, die eine anschließend leicht abspaltbare Gruppe haben, in die Reaktion einzusetzen. Als leicht abspaltbare Gruppe ist beispielsweise die Trialkylsilylgruppe geeignet.

Die Abspaltung erfolgt vor Aufarbeitung des Reaktionsgemisches nach den bekannten Methoden wie beispielsweise durch Zusatz von Basen bei Zimmertemperatur. Geeignete Basen sind zum Beispiel Alkalihydroxide und- alkoholate wie Natrium- oder Kalium-hydroxid, methylat oder- ethylat oder Fluoride wie Cäsiumfluorid oder Tetra-n-butyl-ammonium-fluorid.

Gewünschtenfalls können in die Reaktion auch die in 9-Stellung mit einer Schutzgruppe wie der Tosylgruppe substituierten β-Carboline eingesetzt werden. Diese Schutzgruppe wird wie oben beschrieben bei der Aufarbeitung des Reaktionsgemisches oder anschließend durch Behandeln mit Alkalialkoholaten abgespalten.

Werden die erfindungsgemäßen Verbindungen nach der Verfahrensvariante c) hergestellt, so kann man beispielsweise nachdem von J. Lin, S.A. Lang, J. Org. Chem. 1980, 4857 beschriebnen Verfahren vorgehen, indem man zunächst das Enaminon bildet, das im allgemeinen ohne Aufarbeitung mit Hydroxylamin-O-sulfonsäure cyclisiert wird. Die Umsetzung wird bei Raumtemperatur bis zu 100°C mit oder ohne Lösungsmittelzusatz durchgeführt. Zur Enaminon-Bildung setzt man beispielsweise Dialkylformamiddialkylacetal oder Aminalester ein. Die Cyclisierung kann in protischen Lösungsmitteln wie Alkoholen beispielsweise Methanol, Ethanol, Propanol u.a. vorgenommen werden und ist nach 1-24 Stunden beendet.

Die Herstellung der Ausgangsverbindungen ist bekannt oder erfolgt nach bekannten Verfahren.

Beispielsweise erfolgt die Herstellung der Nitriloxide durch Abspaltung von Halogenwasserstoff aus den Hydroxamsäurehalogeniden mit Basen wie Natrium- oder Kaliumalkoholaten, Trialkylaminen, Hünig Base, DBU oder Diazabicyclooctan bei Raumtemperatur. Die Hydroxamsäurehalogenide erhält man beispielsweise durch Umsetzung der entsprechenden Oxime mit N-Brom-succinimid, N-Chlor-succinimid, tert. Butoxychlorit oder Na-hypohalogenit in den vorher genannten aprotischen Lösungsmitteln (R. Annunziata et al., J. chem. Soc. Chem. Comm. 1987, 529, K. Larsen et al. Tetr. 1984, 2985).

Nitriloxide können auch durch formale Wasserabspaltung aus den entsprechenden Nitroverbindungen durch Umsetzung mit einem Säurechlorid oder Arylisocyanat in Gegenwart einer Base wie Trialkylamin oder Alkalialkoholat in den oben genannten aprotischen Lösungsmitteln bei Temperaturen von -10°C bis 40°C erhalten werden (K. Harada et al. Chem. Pharm. Bull. Jpn 1980, 3296, H. Kawakami et al. Chem. Lett. 1987, 85).

Die Herstellung von Ausgangsmaterial ist beispielsweise in der EP-A 54 507, EP-A 218541, EP-A 130 140 und EP-A 237467 beschrieben.

Die nachfolgenden Beispiele sollen das erfindungsgemäße Verfahren erläutern.

### Herstellung der Ausgangsverbindungen

6-Benzyloxy-4-methoxymethyl-β-carbolin-3-carbaldehyd
Zu einer Suspension von 24,3 g (60 mmol) 6-Benzyloxy-4-methoxymethyl-β-carbolin-3-carbonsäure-isopropylester in 250 ml trockenem Toluol werden 15 ml Chlortrimethylsilan und 48 ml Triethylamin hinzugefügt. Die Reaktionsmischung wird eine Stunde auf 50°C erwärmt und dann auf das halbe Volumen eingeengt. Danach wird das Reaktionsgemisch auf -78°C abgekühlt und unter Stickstoff innerhalb von 1/2 Stunde werden 100 ml DIBAH (1M-Lösung in Toluol) zugefügt und es wird für eine weitere halbe Stunde bei -78°C gerührt. Dann wird mit 10 ml Ethanol versetzt und auf Zimmertemperatur erwärmt, 200 ml 0,5 M-Natriumhydroxidlösung und 200 ml werden Ethanol zugegeben und es wird über Nacht gerührt. Der Niederschlag wird abfiltriert, mit Wasser gewaschen, getrocknet und ohne weitere Reinigung in den nächsten Reaktionsschritt eingesetzt.

6-Benzyloxy-4-methoxymethyl-β-carbolin-3-carbaldehyd-oxim
13,5 g 6-Benzyloxy-4-methoxymethyl-β-carbolin-3-carbaldehyd werden in 150 ml trockenem DMF gelöst und 6 g Hydroxylamin-Hydrochlorid und eine Lösung von 6 g Kaliumhydroxid in 30 ml Ethanol hinzugefügt. Das Reaktionsgemisch wird eine Stunde bei Raumtemperatur gerührt, filtriert und der Rückstand mit 2x20 ml DMF gewaschen. Zu der DMF-Fraktion werden 200 ml Eiswasser gegeben, der Niederschlag abfiltriert, mit Wasser gewaschen und getrocknet.
Ausbeute 9,6 g
Analog werden hergestellt:
6-(2-Pyrazinyloxy)-4-methoxymethyl-β-carbolin-3-carbaldehydoxim
6-(4-Chlorphenoxy)-4-methoxymethyl-β-carbolin-3-carbaldehydoxim
6-(2-Pyridyloxy)-4-methoxymethyl-β-carbolin-3-carbaldehydoxim
6-Methyl-4-methoxymethyl-β-carbolin-3-carbaldehydoxim
6-(5-Brom-pyrid-2-yl)-4-methoxymethyl-β-carbolin-3-carbaldehydoxim
6-Benzyloxy-4-methyl-β-carbolin-3-carbaldehydoxim
5-Benzyloxy-4-methoxymethyl-9-tosyl-β-carbolin-3-carbaldehydoxim
500 mg 5-Benzyloxy-4-methoxymethyl-9-tosyl-β-carbolin-3-carbaldehyd werden in 15 ml Ethanol auf 70°C erwärmt, mit 84 mg Hydroxylaminhydrochlorid in 10 ml Ethanol versetzt und 1,5 Stunden auf 70°C erwärmt. Anschließend wird auf 5 ml eingeengt auf 40 ml Wasser gegeben und abgesaugt. Der Rückstand (450 mg) wird nach Trocknen ohne weitere Reinigung in die nächste Stufe eingesetzt.

In analoger Weise werden hergestellt:
5-Isopropoxy-4-methyl-9-tosyl-β-carbolin-3-carbaldehydoxim
5-(4-Chlorphenoxy)-4-methoxymethyl-9-tosyl-β-carbolin-3-carbaldehydoxim
5-(2-Pyrazinyloxy)-4-methoxymethyl-9-tosyl-β-carbolin-3-carbaldehydoxim
5-(5-Brom-2-pyridinyloxy)-4-methoxymethyl-9-tosyl-β-carbolin-3-carbaldehydoxim
6,7-Dimethoxy-4-ethyl-9-tosyl-β-carbolin-3-carbaldehydoxim
3-Acetyl-5-benzyloxy-4-methoxymethyl-9-tosyl-β-carbolin
3,3 g 5-Benzyloxy-4-methoxymethyl-9-tosyl-β-carbolin-3-carbonsäureethylester werden in 25 ml abs. THF suspendiert und unter N₂-Atmospäre auf -60°C, gekühlt. Zu dieser Suspension werden 5,2 ml einer 1,5 m etherischen Methyllithiumlösung getropft und 2 Stunden bei -60°C nachgerührt. Nach Erwärmen auf Raumtemperatur wird das Reaktionsgemisch mit gesättigter Ammoniumchloridlösung versetzt und mit Essigester extrahiert. Das Rohprodukt wird mittels Chromatographie an Kieselgel mit Toluol + Essigester = 95 + 5 gereinigt.

3-Acetyl-6-benzyloxy-4-methoxymethyl-β-carbolin
Zu 3,70 g (22 mmol) Methansulfin-p-toluidid in 100 ml trockenem THF werden bei -78°C unter N₂-Atmospähre 45 mmol n-Butyllithium innerhalb von 10 Minuten getropft. 5,58 g (10 mmol) 6-Benzyloxy-4-methoxymethyl-9-tosyl-β-carbolin-3-carbonsäureisopropylester gelöst in 50 ml trockenem THF werden hinzugefügt. Das Reaktionsgemisch wird dunkelblau. Es wird 1 Stunde bei -78°C nachgerührt, in Wasser gegossen und mit Ether extrahiert. Der Ether wird abgezogen und das zurückbleibende Öl in 100 ml Methanol gelöst. Dann werden 5 g KOH zugegeben und 1 Stunde unter Rückfluß gerührt. Nach dem Abkühlen wird Eiswasser zugegeben und der Niederschlag abfiltriert. Ausbeute 3,5 g.

Das Rohprodukt wird mittels Chromatographie an Kieselgel mit Essigester gereinigt. Ausbeute 2,80 g.

### Beispiel 1

6-Benzyloxy-3-(3-isoxazolyl)4-methoxymethyl-β-carbolin
0,55 g (1,5 mmol) 6-Benzyloxy-4-methoxymethyl-β-carbolin-3-carbaldehydoxim werden in 30 ml trockenem DMF gelöst und mit 0,3 g (1,7 mmol) N-Brom-succinimid (gelöst in 5 ml DMF) versetzt. Das Reaktionsgemisch wird 10 Minuten bei Raumtemperatur gerührt und dann werden 1,5 eq. Trimethylsilylacetylen und 1 ml Triethylamin hinzugefügt. Nach 4 Stunden Rühren bei Raumtemperatur werden 5 ml 1 M Natriumhydroxydlösung hinzugefügt und anschließend wird eine weitere Stunde gerührt. Die Lösung wird in Eiswasser gegossen und mit Essigester extrahiert. Die organische Phase wird über Magnesiumsulfat getrocknet und das Lösungsmittel abgezogen. Das erhaltene Produkt wird säulenchromatographisch an Kieselgel mit Essigester als Elutionsmittel gereinigt.
Schmelzpunkt: 123-126°C

### Beispiel 2

6-Benzyloxy-3-(5-ethoxy-3-isoxazolyl)-4-methoxymethyl-β-carbolin
1,1 g (3,0 mmol) 6-Benzyloxy-4-methoxymethyl-β-carbolin-3-carbaldehydoxim werden in 40 ml trockenem DMF gelöst und mit 0,56 g (3,1 mmol) N-Brom-succinimid (gelöst in 5 ml DMF) versetzt. Das Reaktionsgemisch wird 15 Minuten bei Raumtemperatur gerührt und dann mit 1,5 eq, Ethoxyacetylen und 2 ml Triethylamin versetzt. Die Reaktionsmischung wird über Nacht bei Raumtemperatur gerührt, anschließend auf Eiswasser gegossen und mit Essigester extrahiert. Die org. Phase wird über Magnesiumsulfat getrocknet, filtriert, das Lösungsmittel abgezogen und der Rückstand säulenchromatographisch an Kieselgel mit Essigester gereinigt.
Schmelzpunkt; 139-140°C
Analog werden hergestellt:
6-Benzyloxy-3-(5-hydroxymethyl-3-isoxazolyl)-4-methoxymethyl-β-carbolin mit Hydroxymethyl-acetylen. Schmelzpunkt: 220-221°C
6-Benzyloxy-3-(5-methoxymethyl-3-isoxazolyl)-4-methoxymethyl-β-carbolin mit Methoxymethyl-acetylen. Schmelzpunkt: 89-90°C
Mit Propargylsäureethylester werden säulenchromatographisch 6-Benzyloxy-3-(5-carbethoxy-3-isoxazolyl)-4-methoxymethyl-β-carbolin
Schmelzpunkt: 202-203°C und
6-Benzyloxy-3-(4-carbethoxy-3-isoxazolyl)-4-methoxymethyl-β-carbolin Schmelzpunkt: 145-146°C
erhalten.
6-Benzyloxy-3-(4-carbethoxy-4,5-dihydro-3-isoxazolyl)-4-methoxymethyl-β-carbolin mit Acrylsäureethylester
Schmelzpunkt 183°C
6-Benzyloxy-3-(4-carbethoxy-4,5-dihydro-4-methyl-3-isoxazolyl)-4-methoxymethyl-β-carbolin mit Methacrylsäureethylester
Schmelzpunkt 187-189°C
6-Benzyloxy-3-(5-ethoxy-4,5-dihydro-3-isoxazolyl)-4-methoxymethyl-β-carbolin mit Vinylethylether
Schmelzpunkt: 149-150°C
6-Benzyloxy-3-(5-methyl-3-isoxazolyl)-4-methoxymethyl-β-carbolin
Schmelzpunkt: 160-162°C
6-(2-Pyrazinyloxy)-3-(5-methoxymethyl-3-isoxazolyl)-4-methoxymethyl-β-carbolin
Schmelzpunkt: 184°C
6-Methyl-3-(5-methyl-3-isoxazolyl)-4-methoxymethyl-β-carbolin
Schmelzpunkt: 178°C
6-Methyl-3-(5-methoxymethyl-3-isoxazolyl)-4-methoxymethyl-β-carbolin
Schmelzpunkt: 160°C
6-(5-Brompyrid-2-yl)-oxy-4-methoxymethyl-3-(3-isoxazolyl)-β-carbolin
Schmelzpunkt: 203°C
6-(5-Brompyrid-2-yl)-oxy-3-(5-methyl-3-isoxazolyl)-4-methoxymethyl-β-carbolin
Schmelzpunkt: 197-198°C
6-(5-Brompyrid-2-yl)-oxy-3-(5-methoxymethyl-3-isoxazolyl)-4-methoxymethyl-β-carbolin
Schmelzpunkt: 164°C
6-Benzyloxy-4-methoxymethyl-3-(5-isopropyl-3-isoxazolyl)-β-carbolin
6-Benzyloxy-4-methoxymethyl-3-(5-cyclopentyl-3-isoxazolyl)-β-carbolin
6-Benzyloxy-4-methoxymethyl-3-(5-ethoxymethyl-3-isoxazolyl)-β-carbolin
6-Benzyloxy-4-methyl-3-(5-ethoxymethyl-3-isoxazolyl)-β-carbolin

### Beispiel 3

6-Brom-3-(3-isoxazolyl)-β-carbolin
1,05 g (5 mmol) β-Carbolin-3-carbaldehydoxim werden in 50 ml trockenem THF suspendiert und bei 0°C mit 1,8 g N-Brom-succinimid in 20 ml trockenem THF versetzt. Das Reaktionsgemisch wird auf Raumtemperatur erwärmt, 95 Stunden nachgerührt und mit 1,5 eq. Trimethylsilylacetylen und 2 ml Triethylamin versetzt. Die Mischung wird über Nacht nachgerührt, auf Eiswasser gegossen und mit Ether extrahiert. Die org. Phase wird über Magnesiumsulfat getrocknet, filtriert und das Lösungsmittel abgezogen. Das zurückbleibende Öl wird in 10 ml DHF gelöst und mit 10 ml Natriumhydroxydlösung (1 M) versetzt. Die Reaktionsmischung wird über Nacht bei Raumtemperatur gerührt, auf Wasser gegossen und mit Essigester extrahiert. Das erhaltene Produkt wird säulenchromatographisch mit Essigester als Elutionsmittel gereinigt.
Schmelzpunkt: 292-293°C

### Beispiel 4

5-Benzyloxy-4-methoxymethyl-3-(3-isoxazolyl)-β-carbolin
103 mg 5-Benzyloxy-4-methoxymethyl-9-tosyl-β-carbolin-3-carbaldehydoxim werden in 5 ml Methylenchlorid mit 22 mg N-Chlorsuccinimid versetzt und 1 Stunde bei Raumtemperatur gerührt. Anschließend werden 30 mg Trimethylsilylacetylen und 0,13 ml Triethylamin zugegeben und es wird 2 Stunden bei Raumtemperatur gerührt. Anschließend wird mit 1 ml 1-N-Natronlauge versetzt und 1 Stunde bei Raumtemperatur gerührt, auf 25 ml Wasser gegeben, mit 25 ml Methylenchlorid extrahiert und die organische Phase getrocknet, filtriert und eingeengt. Der Rückstand wird in 15 ml Methanol mit 54 mg Natriummethylat 1,5 Stunden am Rückfluß gekocht. Nach Einengen wird in 25 ml Wasser aufgenommen und mit Essigester extrahiert. Nach Trocknen und Filtrieren wird die organische Phase eingeengt und der Rückstand über Kieselgel mit Toluol:Essigester = 1:1 als Laufmittel chromatographiert.

### Beispiel 5

5-Benzyloxy-4-methoxymethyl-3-(5-methoxymethyl-3-isoxazolyl)-β-carbolin
Zu 155 mg 5-Benzyloxy-4-methoxymethyl-9-tosyl-β-carbolin-3-carbaldoximhydrochlorid in 6 ml absolutem Tetrahydrofuran werden 1,4 ml Natriumhypochloridlösung bei Raumtemperatur unter Schutzgas zugetropft. Man rührt bis das Oxim verschwunden ist (DC Kontrolle) 1 Stunde bei Raumtemperatur, dann werden 210 mg Methylpropargyläther zugetropft und über Nacht bei Raumtemperatur gerührt. Nach Abdestillieren des Lösungsmittels wird in Essigester/Wasser verteilt und die organische Phase über Magnesiiumsulfat getrocknet, filtriert und eingeengt. Der Rücksstand wird in 8 ml Methanol gelöst, mit 54 mg Natriummethylat versetzt und 1 Stunde zum Rückfluß erhitzt. Nach Einengen und Verteilen in Essigester/konz. Kochsalzlösung wird die organische Phase getrocknet, filtriert und eingeengt. Der Rückstand wird über Kieselgel mit Toluol : Essigsester = 1 : 1 chromatographiert. Die entsprechenden Fraktionen werden zusammengefaßt und aus Essigester kristallisiert. Man erhält 70 mg vom Schmelzpunkt 133 - 135°C (Essigester).
Analog werden hergestellt:
5-(4-Chlorphenoxy)-4-methoxymethyl-3-(5-methoxymethyl-3-isoxazolyl)-β-carbolin vom Schmelzpunkt 176-177°C (Isopropanol)
5-Isopropoxy-4-methyl-3-(5-methoxymethyl-3-isoxazolyl)-β-carbolin
6,7-Dimethoxy-4-ethyl-3-(5-methoxymethyl-3-isoxazolyl)-β-carbolin
5-(2-Pyrazinyloxy)-4-methoxymethyl-3-(5-methoxymethyl-3-isoxazolyl)-β-carbolin
5-(5-Brom-2-pyridinyloxy)-4-methoxymethxyl-3-(5-methoxymethyl-3-isoxazolyl)-β-carbolin

### Beispiel 6

6-Benzyloxy-3-(3-methyl-5-isoxazolyl)-4-methoxymethyl-β-carbolin
340 mg Nitroethan werden in 20 ml trockenem Dimethylacetamid mit 4,5 ml methanolischer 1 N Natriummethylatlösung versetzt und im Eisbad auf 5°C gekühlt. Hierzu werden nacheinander 0,32 ml Acetylchlorid und 510 ml 6-Benzyloxy-4-methoxy-methyl-3-ethinyl-β-carbolin gegeben.

Nach 16 Stunden Rühren bei Raumtemperatur werden weitere 3 Equivalente methanolischer 1 N Natriummethylatlösung, Nitroethan und Acetylchlorid zugegeben und abermals 16 Stunden bei Raumtemperatur gerührt. Diese Addition wird wiederholt, und nach insgesamt 3 Tagen werden 100 ml Wasser zugegeben und nochmals über Nacht bei Raumtemperatur gerührt. Das Reaktionsgemisch wird mit Essigester extrahiert. Die organische Phase wird abgetrennt, getrocknet, filtriert und eingeengt.

Nach Chromatographie über Kieselgel mit Essigester erhält man 130 ml 6-Benzyloxy-3-(3-methy-5-isoxazolyl)-4-methoxymethyl-β-carbolin vom Schmelzpunkt 202-203°C.

### Beispiel 7

6-Benzyloxy-4-methoxymethyl-3-(5-isoxazolyl)-β-carbolin
500 mg 3-Acetyl-6-benzyloxy-4-methoxymethyl-β-carbolin werden in 5 ml N,N-Dimethylformamiddiethylacetal bei 100°C über Nacht gerührt. Nach Eindampfen wird ohne weitere Reinigung in 20 ml absolutem Ethanol aufgenommen mit 600 mg Hydroxylamin-O-Sulfonsäure in 5 ml Methanol versetzt und für 5 Stunden bei Raumtemperatur gerührt. Nach Neutralisation mit Triethylamin wird das Reaktionsgemisch über Nacht gerührt, in Wasser eingetragen und mit Essigester extrahiert. die organische Phase wird getrocknet über Magnesiumsulfat und im Vakuum eingeengt. Nach Chromatographie über Kieselgel mit Aceton/Triethylamin = 10:1 als Elutionsmittel erhält man 130 mg 6-Benzyloxy-4-methoxymethyl-3-(5-isoxazolyl)-β-carbolin vom Schmelzpunkt 125-126°C.

## Patentansprüche

1. Verbindungen der allgemeinen Formel I 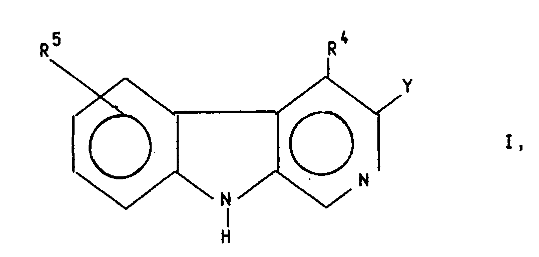 worin
Y den Rest 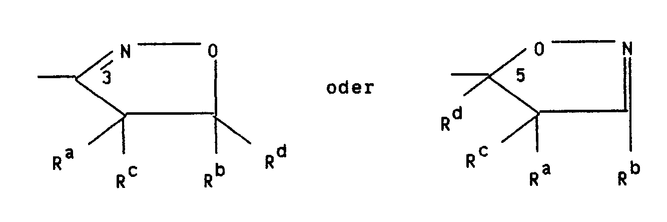 darstellt und
R^{a} und R^{b} gleich oder verschieden sind und jeweils Wasserstoff, C₁₋₆-Alkoxy, C₃₋₇-Cycloalkyl, Phenyl, gegebenenfalls mit Hydroxy, C₁₋₆-Alkoxy, Phenyl oder Halogen substituiertes C₁₋₆-Alkyl oder C₁₋₆-Alkoxycarbonyl darstellen und R^{c} und R^{d} gleich oder verschieden sind und jeweils Wasserstoff oder C₁₋₆-Alkyl oder gemeinsam eine Bindung bedeuten und
R⁴ Wasserstoff, C₁₋₆-Alkyl oder C₁₋₆ -Alkoxy-C₁₋₆-Alkyl ist und
R⁵ Wasserstoff, Halogen, OR⁶, NR⁷R⁸ oder CH R⁹R¹⁰ ist
mit R⁶ in der Bedeutung von C₁₋₆-Alkyl, C₃₋₇ Cycloalkyl, eines gegebenenfalls mit Halogen, C₁₋₄-Alkoxy, C₁₋₄-Alkyl oder Amino ein- oder zweifach substituierten C₇₋₁₀-Aralkylreste, eines gegebenenfalls mit Halogen, NO₂, Cyano, C₁₋₄-Alkyl oder C₁₋₄-Alkoxy ein- bis zweifach substituiertes C₆₋₁₀-Arylrestes, eines gegebenenfalls mit Halogen, NO₂, Cyano, C₁₋₄-Alkyl oder C₁₋₄-Alkoxy ein- bis dreifach substituierten Sechsring-Heteroaromaten mit ein- bis zwei Stickstoffatomen oder eines gegebenenfalls mit Halogen, NO₂, Cyano, C₁₋₄-Alkyl oder C₁₋₄-Alkoxy ein- bis dreifach substituierten Fünfringheteroaromaten mit ein- bis zwei Sauerstoff-, Schwefel- und/oder Stickstoffatomen, R⁷ und R⁸ sind gleich oder verschieden und stellen Wasserstoff, C₁₋₆-Alkyl, C₃₋₆-Alkenyl oder gemeinsam mit dem Stickstoffatom einen gegebenenfalls mit ein bis zwei C₁₋₄-Alkylgruppen substituierten Pyrrolidin-, Piperidin-, Morpholin-, Piperazin- oder Thiomorpholin-Ringdar, R⁹ in der Bedeutung von Wasserstoff oder C₁₋₆-Alkyl, R¹⁰ in der Bedeutung von Wasserstoff, C₁₋₆-Alkyl, OR¹¹ oder NR¹²R¹³ mit R¹¹ in der Bedeutung von C₁₋₆ Alkyl, R¹² und R¹³ sind gleich oder verschieden und stellen Wasserstoff, C₁₋₆-Alkyl oder gemeinsam mit dem Stickstoffatom einen gegebenenfalls mit ein bis zwei C₁₋₄-Alkylgruppen substituierten Pyrrolidin-, Piperidin-, Morpholin-, Piperazin- oder Thiomorpholin-Ringdar und der Substituent R⁵ ein- oder zweifach steht, wobei, falls R⁴ und R⁵ Wasserstoff bedeuten, Y nicht 3-Methyl-isoxazol-5-yl sein kann.

2. 6-Benzyloxy-3-(3-isoxazolyl)-4-methoxymethyl-(β-carbolin
6-Benzyloxy-3-(5-ethoxy-3-isoxazolyl)-4-methoxymethyl-β-carbolin
6-Benzyloxy-3-(5-hydroxymethyl-3-isoxazolyl)-4-methoxymethyl-β-carbolin
6-Benzyloxy-3-(5-methoxymethyl-3-isoxazolyl)-4-methoxymethyl-β-carbolin
6-Benzyloxy-3-(5-ethoxy-4,5-dihydro-3-isoxazolyl)-4-methoxymethyl-β-carbolin
6-Benzyloxy-3-(5-methyl-3-isoxazolyl)-4-methoxymethyl-β-carbolin
6-Benzyloxy-4-methoxymethyl-3-(3-methyl-5-isoxazolyl)-β-carbolin
5-Benzyloxy-4-methoxymethyl-3-(5-methoxymethyl-3-isoxazolyl)-β-carbolin
5-(4-Chlorphenoxy)-3-(5-methoxymethyl-3-isoxazolyl)-4-methoxymethyl-β-carbolin
6-(2-Pyrazinyloxy)-4-methoxymethyl-3-(5-methoxymethyl-3-isoxazolyl)-β-carbolin
6-(5-Brompyrid-2-yl-oxy)-4-methoxymethyl-3-(5-methyl-3-isoxazolyl)-β-carbolin
nach Anspruch 1.

3. Arzneimittel auf Basis der Verbindungen nach den Ansprüchen 1 und 2.

4. Verfahren zur Herstellung der Verbindungen gemäß Formel I dadurch, daß man
a) Nitriloxide der allgemeinen Formel II 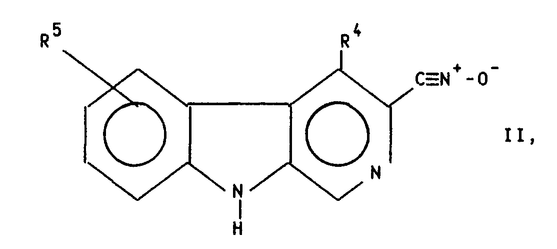 worin
R⁴ und R⁵ die oben genannte Bedeutung haben und der Stickstoff in 9-Stellung geschützt sein kann, mit einer Verbindung der allgemeinen Formel III 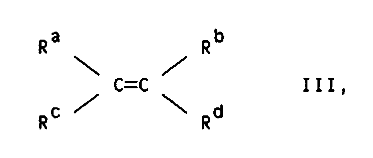 worin R^{a}, R^{b}, R^{c} und R^{d} die obige Bedeutung haben, zu Verbindungen der allgemeinen Formel I mit Y in der Bedeutung 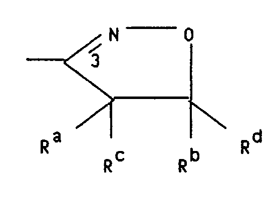 mit R^{a}, R^{b}, R^{c}, und R^{d} in der obigen Bedeutung bei Temperaturen von 0° - 40° C cyclisiert, oder
b) Nitriloxide der allgemeinen Formel IV
R^{b}-C≡N⁺-O⁻ IV,
worin R^{b} die obige Bedeutung hat, mit Verbindungen der allgemeinen Formel V 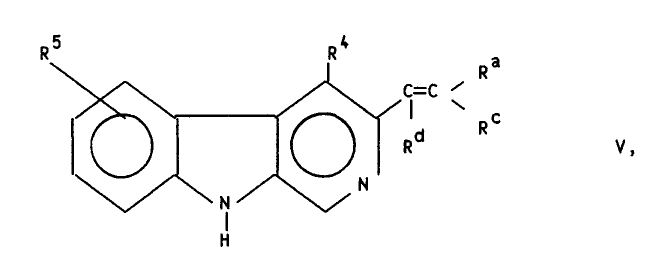 worin
R^{a}, R^{c}, R^{d}, R⁴ und R⁵ die obige Bedeutung haben, zu Verbindungen der allgemeinen Formel I mit Y in der Bedeutung 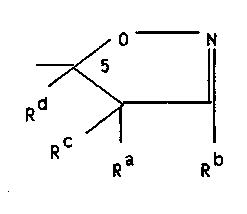 bei Temperaturen von 0° - 40° C cyclisiert, oder
c) Verbindungen der allgemeinen Formel VI 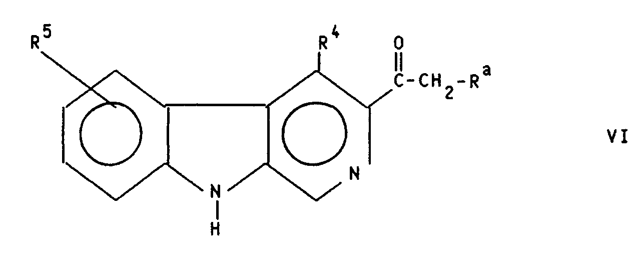 worin
R^{a}, R⁴ und R⁵ die obige Bedeutung haben, zum Enaminon umsetzt und mit Hydroxylamin-O-sulfonsäure bei Raumtemperatur bis zu 100° C cyclisiert zu Verbindungen der allgemeinen Formel I mit Y in der Bedeutung 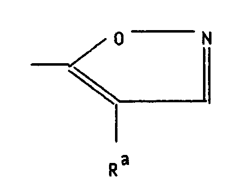 worin R^{a} die obige Bedeutung hat.

5. Verfahren zur Herstellung von Arzneimitteln nach Anspruch 3, dadurch daß man die nach dem Anspruch 4 hergestellten Verbindungen mit üblichen pharmazeutischen Trägern mischt.

## Claims

1. Compounds of the general formula I 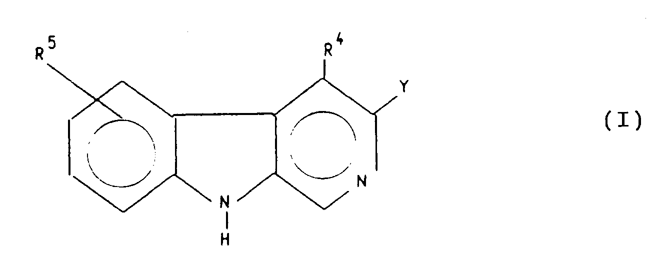 in which
Y represents the radical 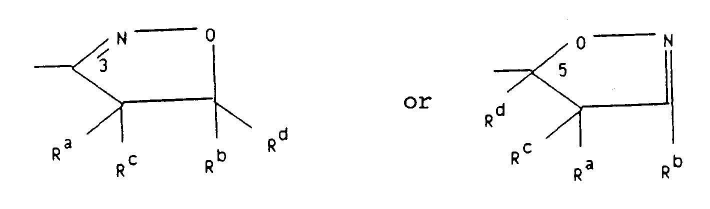 and
R^{a} and R^{b} are the same or different and each represents hydrogen, C₁₋₆-alkoxy, C₃₋₇-cycloalkyl, phenyl, C₁₋₆-alkyl optionally substituted by hydroxy, C₁₋₆-alkoxy, phenyl or by halogen, or represents C₁-₆-alkoxycarbonyl, and R^{c} and R^{d} are the same or different and each represents hydrogen or C₁₋₆-alkyl or together form a bond, and R⁴ is hydrogen, C₁₋₆-alkyl or C₁₋₆-alkoxy-C₁₋₆-alkyl, and R⁵ is hydrogen, halogen, OR⁶, NR⁷R⁸ or CHR⁹R¹⁰, wherein R⁶ represents C₁₋₆-alkyl, C₃₋₇-cycloalkyl, a C₇₋₁₀-aralkyl radical optionally mono- or di-substituted by halogen, C₁₋₄-alkoxy, C₁₋₄-alkyl or by amino, a C₆₋₁₀-aryl radical optionally mono- or di-substituted by halogen, NO₂, cyano, C₁₋₄-alkyl or by C₁₋₄-alkoxy, a 6-membered heteroaromatic ring having one or two nitrogen atoms that is optionally mono- to tri-substituted by halogen, NO₂, cyano, C₁₋₄-alkyl or by C₁₋₄-alkoxy, or a 5-membered heteroaromatic ring having one or two oxygen, sulphur and/or nitrogen atoms that is optionally mono- to tri-substituted by halogen, NO₂, cyano, C₁₋₄-alkyl or by C₁₋₄-alkoxy, R⁷ and R⁸ are the same or different and represent hydrogen, C₁₋₆-alkyl or C₃₋₆-alkenyl or, together with the nitrogen atom, form a pyrrolidine, piperidine, morpholine, piperazine or thiomorpholine ring that is optionally substituted by one or two C₁₋₄-alkyl groups, R⁹ represents hydrogen or C₁₋₆-alkyl, R¹⁰ represents hydrogen, C₁₋₆-alkyl, OR¹¹ or NR¹²R¹³ wherein R¹¹ represents C₁₋₆-alkyl, R¹² and R¹³ are the same or different and represent hydrogen or C₁₋₆-alkyl or, together with the nitrogen atom, form a pyrrolidine, piperidine, morpholine, piperazine or thiomorpholine ring that is optionally substituted by one or two C₁₋₄-alkyl groups, and the substituent R⁵ is present once or twice, with the proviso that Y may not be 3-methyl-isoxazol-5-yl when R⁴ and R⁵ are hydrogen.

2. 6-Benzyloxy-3-(3-isoxazolyl)-4-methoxymethyl-β-carboline,
6-benzyloxy-3-(5-ethoxy-3-isoxazolyl)-4-methoxymethyl-β-carboline,
6-benzyloxy-3-(5-hydroxymethyl-3-isoxazolyl)-4-methoxymethyl-β-carboline,
6-benzyloxy-3-(5-methoxymethyl-3-isoxazolyl)-4-methoxymethyl-β-carboline,
6-benzyloxy-3-(5-ethoxy-4,5-dihydro-3-isoxazolyl)-4-methoxymethyl-β-carboline,
6-benzyloxy-3-(5-methyl-3-isoxazolyl)-4-methoxymethyl-β-carboline,
6-benzyloxy-4-methoxymethyl-3-(3-methyl-5-isoxazolyl)-β-carboline,
5-benzyloxy-4-methoxymethyl-3-(5-methoxymethyl-3-isoxazolyl)-β-carboline,
5-(4-chlorophenoxy)-3-(5-methoxymethyl-3-isoxazolyl)-4-methoxymethyl-β-carboline,
6-(2-pyrazinyloxy)-4-methoxymethyl-3-(5-methoxymethyl-3-isoxazolyl)-β-carboline,
6-(5-bromopyrid-2-yl-oxy)-4-methoxymethyl-3-(5-methyl-3-isoxazolyl)-β-carboline,
according to claim 1.

3. Medicaments based on the compounds according to claims 1 and 2.

4. Process for the preparation of the compounds according to formula I by
a) cyclising nitrile oxides of the general formula II 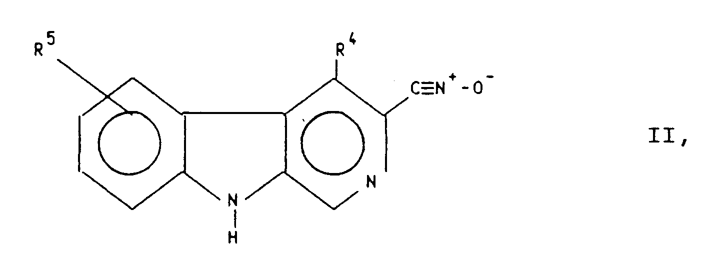 in which R⁴ and R⁵ have the meaning given above and the nitrogen in the 9-position may be protected, with a compound of the general formula III 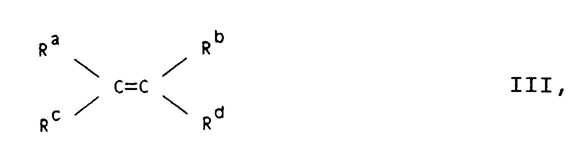 in which R^{a}, R^{b}, R^{c} and R^{d} have the above meaning, at temperatures of from 0° to 40°C, to give compounds of the general formula I wherein Y represents 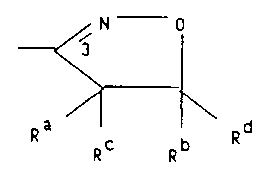 wherein R^{a}, R^{b}, R^{c} and R^{d} have the above meaning, or
b) cyclising nitrile oxides of the general formula IV
R^{b}-C≡N⁺-O⁻ IV,
in which R^{b} has the above meaning, with compounds of the general formula V 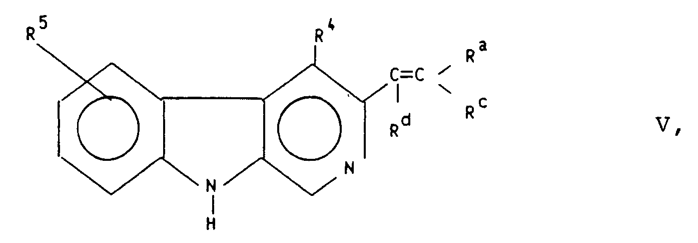 in which R^{a}, R^{c}, R^{d}, R⁴ and R⁵ have the above meaning, at temperatures of from 0° to 40°C, to give compounds of the general formula I wherein Y represents 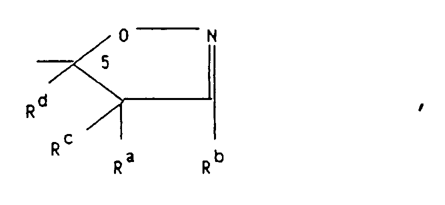 or
c) reacting compounds of the general formula VI 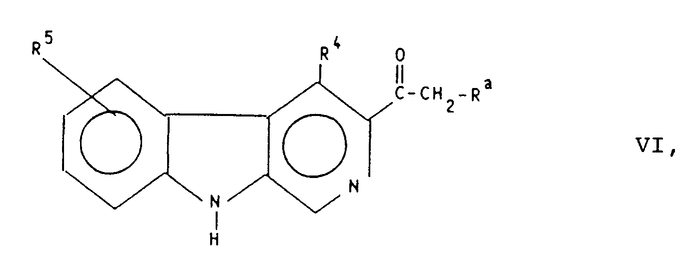 in which R^{a}, R⁴ and R⁵ have the above meaning, to form the enaminone and cyclising the latter with hydroxylamine-O-sulphonic acid at from room temperature to 100°C to give compounds of the general formula I wherein Y represents 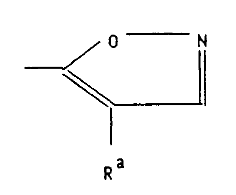 wherein R^{a} has the above meaning.

5. Process for the preparation of medicaments according to claim 3 by mixing the compounds prepared according to claim 4 with customary pharmaceutical carriers.

## Revendications

1. Composés de formule générale I ci-dessous : 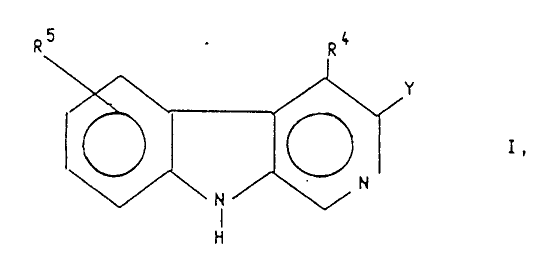 dans laquelle Y représente un radical 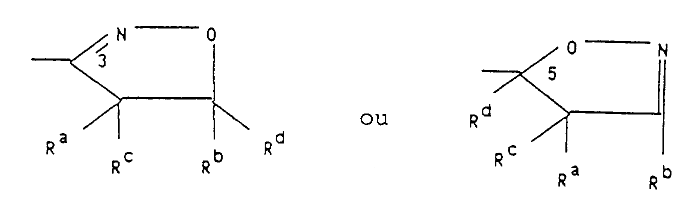 R^{a} et R^{b}, qui peuvent être identiques ou différents l'un de l'autre, étant chacun l'hydrogène, un alcoxy en C₁-C₆, un cycloalkyle en C₃-C₇, un phényle, un alkyle en C₁-C₆ éventuellement substitué par un hydroxyle, un alcoxy en C₁-C₆, un phényle ou un halogène ou bien alcoxycarbonyle en C₁-C₆, et R^{c} et R^{d}, qui peuvent être aussi identiques ou différents l'un de l'autre, étant chacun l'hydrogène ou un alkyle en C₁-C₆ ou bien formant ensemble une liaison,
R⁴ représente l'hydrogène, un alkyle en C₁-C₆ ou un C₁-C₆-alcoxy-C₁-C₆-alkyle et
R⁵ l'hydrogène, un halogène ou un radical OR⁶, NR⁷R⁸ ou CH R⁹R¹⁰
R⁶ désignant un alkyle en C₁-C₆, un cycloalkyle en C₃-C₇, un aralkyle en C₇-C₁₀ avec éventuellement un ou deux substituants pris parmi les halogènes, des alcoxy et alkyles en C₁-C₄ et le groupe amino, un aryle en C₆-C₁₀, avec éventuellement un ou deux substituants pris parmi les halogènes, les groupes NO₂ et cyano et des alkyles et alcoxy en C₁-C₄, ou encore un radical hétéroaromatique à six chaînons avec de un à trois substituants pris parmi les halogènes, les groupes NO₂ et cyano et des alkyles et alcoxy en C₁-C₄ et un ou deux atomes d'azote, ou un radical hétéroaromatique à cinq chaînons avec éventuellement de un à trois substituants pris aussi parmi les halogènes, les groupes NO₂ et cyano et des alkyles et alcoxy en C₁-C₄ et un ou deux atomes d'oxygène, de soufre et/ou d'azote, R⁷ et R⁸, qui peuvent être identiques ou différents l'un de l'autre, désignant l'hydrogène, des alkyles en C₁-C₆ ou des alcényles en C₃-C₆ ou bien formant ensemble et avec l'atome d'azote un cycle de pyrrolidine, de pipéridine, de morpholine, de pipérazine ou de thiomorpholine avec éventuellement comme substituants un ou deux alkyles en C₁-C₄, R⁹ désignant l'hydrogène ou un alkyle en C₁-C₆ et R¹⁰ l'hydrogène, un alkyle en C₁-C₆ ou un radical OR¹¹ ou NR¹²R¹³_{,} R¹¹ étant un alkyle en C₁-C₆ et R¹² et R¹³, identiques ou différents l'un de l'autre, désignant l'hydrogène ou un alkyle en C₁-C₆ ou bien formant ensemble et avec l'atome d'azote un cycle de pyrrolidine, de pipéridine, de morpholine, de pipérazine ou de thiomorpholine avec éventuellement chacun comme substituants un ou deux alkyles en C₁-C₄, et il peut y avoir un ou deux substituants R⁵ et si R⁴ et R⁵ sont l'hydrogène Y ne peut être un radical 3-méthyl-isoxazol-5-yle.

2. 6-Benzyloxy-3-(3-isoxazolyl)-4-méthoxyméthyl-β-carboline
6-Benzyloxy-3-(5-éthoxy-3-isoxazolyl)-4-méthoxyméthyl-β-carboline
6-Benzyloxy-3-(5-hydroxyméthyl-3-isoxazolyl)-4-méthoxyméthyl-β-carboline
6-Benzyloxy-3-(5-méthoxyméthyl-3-isoxazolyl)-4-méthoxyméthyl-β-carboline
6-Benzyloxy-3-(5-éthoxy-4,5-dihydro-3-isoxazolyl)-4-méthoxyméthyl-β-carboline
6-Benzyloxy-3-(5-méthyl-3-isoxazolyl)-4-méthoxyméthyl-β-carboline
6-Benzyloxy-4-méthoxyméthyl-3-(3-méthyl-5-isoxazolyl)-β-carboline
5-Benzyloxy-4-méthoxyméthyl-3-(5-méthoxyméthyl-3-isoxazolyl)-β-carboline
5-(4-Chlorophénoxy-3-(5-méthoxyméthyl-3-isoxazolyl)-4-méthoxyméthyl-β-carboline
6-(2-Pyrazinyloxy)-4-méthoxyméthyl-3-(5-méthoxyméthyl-3-isoxazolyl)-β-carboline
6-(5-Bromopyrid-2-yl-oxy)-4-méthoxyméthyl-3-(5-méthyl-3-isoxazolyl)-β-carboline, selon la revendications 1.

3. Médicaments à base des composés des revendications 1 et 2.

4. Procédé de préparation des composés de formule I suivant lequel :
a) on cyclise à des températures de 0 à 40 °C des oxydes de nitriles de formule II : 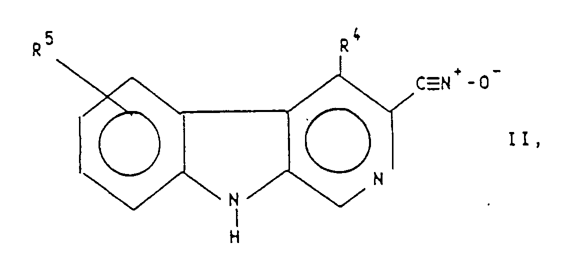 dont l'azote à la position 9 peut être protégé avec un composé de formule III : 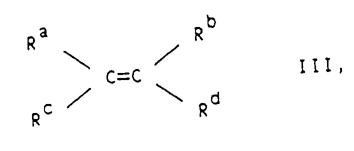 en composés de formule générale I dont Y est un radical : 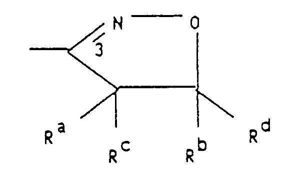 ou bien
b) on cyclise à des températures de 0 à 40 °C des oxydes de nitriles de formule IV :
R^{b}-C≡N⁺-O⁻ IV,
avec des composés de formule V : 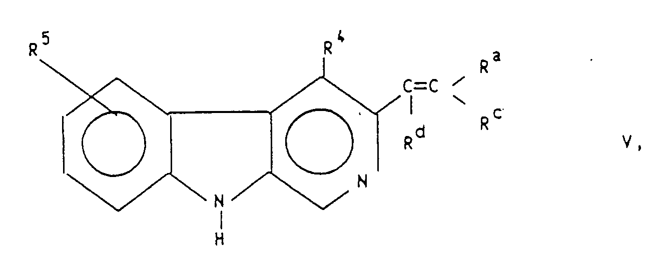 en composés de formule I dont Y est un radical : 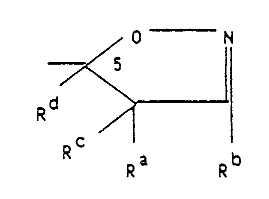 ou encore
c) on transforme des composés de formule VI : 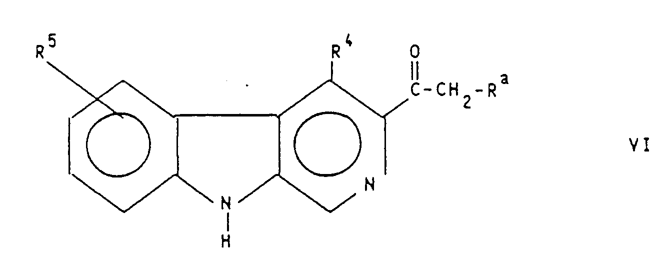 en énaminone et on cyclise avec l'acide hydroxylamino-O-sulfonique, à une température comprise entre la température ambiante et 100 °C, en composés de formule I dont Y est un radical :

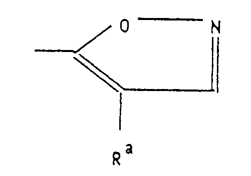

5. Procédé de préparation de médicaments selon la revendication 3 par mélange avec des supports et véhicules pharmaceutiques courants des composés qui ont été obtenus suivant la revendication 4.
